# EUROPEAN PATENT APPLICATION

(11) **EP 4 509 067 A2**
(43) Date of publication of application: **19.02.2025**
(21) Application number: 24194983.3
(22) Date of filing: 16.08.2024
(51) Int. Cl.: A61B 17/15, A61B 17/17, A61B 34/20, A61B 90/00

(54) **PATIENT-SPECIFIC SURGICAL GUIDES**

(30) Priority: 17.08.2023 US 202363520227 P; 08.12.2023 US 202318568378; 09.08.2024 US 202418799385
(71) Applicant: Wright Medical Technology, Inc., Memphis, TN 38117 (US)
(72) Inventor: STEMNISKI, Paul M., Memphis, 38122 (US); MOORE, Jesse G., Germantown, 38138 (US); KEMPER, Jakob, Heemstede (NL)
(74) Representative: Zimmermann & Partner Patentanwälte mbB

(57) **Abstract**

Provided is a method for a surgical procedure on a bone of a patient that utilizes a guide assembly formed of at least one reusable component that contains a plurality of radio-opaque markers representing the guide assembly's orientation and position with respect to the bone when viewed with an X-ray imaging tool.

## Description

### CROSS-REFERENCE TO RELATED APPLICATIONS

This application is a continuation-in-part of co-pending U.S. patent Application No. 17/663,730, filed May 17, 2022, which claims priority to U.S. Provisional Applications No. 63/223,224, filed July 19, 2021, and No. 63/201,950, filed May 20, 2021. This application is also a continuation-in-part of co-pending U.S. patent Application No. 18/568,378, filed December 8, 2023, which is a §371 application of PCT/IB2021/055022, filed June 8, 2021. This application also claims priority under 35 U.S.C. § 119(e) to U.S. Provisional Application No. 63/520,227, filed August 17, 2023. The entire contents of the above-referenced patent applications are incorporated herein by reference.

### FIELD

This disclosure relates to surgical devices and methods of manufacture and use thereof. More specifically, the disclosure relates to guide systems with components having at least one surface that matches a profile of a target area, for example, a bone surface of a patient, and the manufacturing methods of the components of the guide system and use thereof in surgical procedures.

### BACKGROUND

Joint replacement surgeries are complicated and time consuming. Generally, any steps removed or combined may lead to a faster surgical time, increased customer satisfaction, and/or reduced risk for the patient. Joint replacement surgery with patient-matched (also referred to as patient-specific) instruments may remove alignment steps. However, patient-matched components are generally manufactured using plastic which is not always desired because of the possibility of plastic debris being generated during surgical procedures. Thus, when patient-matched structures are incorporated into guiding instruments for surgical procedures such as drilling, cutting, reaming, etc., a separate metallic component is often used in combination with the patient-matched plastic structure as protective sleeves, liners, or inserts.

Although the patient-matched feature (e.g., the patient-matched surface) of the patient-matched instruments are intended to facilitate proper placement of the patient-matched instruments in the patient's joint, in some situations some abnormal conditions on the periosteum, such as a bump, can interfere with the desired seating of the patient-matched instrument. Thus, additional means of verifying the proper alignment and placement of the patient-matched instrument via fluoroscope is desired.

### SUMMARY

Provided is a method for a surgical procedure on a bone of a patient, the method comprising:
(a) positioning a patient-matched guide body on the bone, wherein the patient-matched guide body comprises a recess configured to receive an operational jig;
(b) inserting the operational jig into the recess in the patient-matched guide body thus forming a guide assembly that comprises the patient-matched guide body and the operational jig, wherein the operational jig or the patient-matched guide body includes a plurality of radio-opaque markers representing the guide assembly's orientation and position with respect to the bone when viewed with an X-ray imaging tool of a surgical system; and
(c) projecting, on the surgical system's display screen, a visual measure of quality of the guide assembly's placement and alignment on the bone.

Also provided is a surgical system comprising:
an X-ray imaging device;
a display screen; and
a computing device configured to display an X-ray image of a bone of a patient on which a patient-matched guide assembly is positioned, on the display screen;
   wherein the patient-matched guide assembly includes a plurality of radio-opaque markers representing the patient-matched guide assembly's placement and alignment with respect to the bone when viewed with the X-ray imaging device;
   wherein the computing device is further configured to project, on the display screen, a visual measure of quality of the patient-matched guide assembly's placement and alignment on the bone.

Also provided is a surgical kit comprising:
a guide assembly for attaching to a patient's bone to guide bone resection procedures, the guide assembly comprises:
an operational jig that comprises: a plurality of radio-opaque markers; one or more cutting guide slots; and one or more drill guide holes, wherein the radio-opaque markers representing the guide assembly's orientation and position with respect to the bone when viewed with an X-ray imaging tool; and
a patient-matched guide body that comprises: a first side; a second side opposite the first side; and a guide receptacle for receiving the operational jig, wherein the second side includes at least one patient-specific surface that is configured for complementarily engaging the anatomical surface features of a target area of a patient's bone.

Further disclosed herein is a method for a surgical procedure on a bone of a patient that utilizes a guide assembly formed of at least one reusable component that contains a plurality of radio-opaque markers representing the guide assembly's orientation and position with respect to the bone when viewed with an X-ray imaging tool.

### BRIEF DESCRIPTION OF THE DRAWINGS

These and other features and advantages of the guide assembly and methods of using the guide assembly described herein will be more fully disclosed in the following detailed description of the preferred embodiments, which is to be considered together with the accompanying drawings wherein like numbers refer to like parts and further wherein:
FIG. 1A is an illustration of a guide assembly according to an embodiment of the present disclosure.
FIG. 1B is an illustration of a patient-matched guide body according to an embodiment of the present disclosure.
FIG. 1C is an illustration of an operational jig according to an embodiment of the present disclosure.
FIG. 1D is an illustration showing the patient-matched guide body and an operational jig being assembled together to form a guide assembly of the present disclosure.
FIG. 2A is an example of a fluoroscopic image taken in a frontal plane (Anterior-Posterior view) that may be displayed to a user during a surgical procedure showing the divergence between the intended target position of a guide body and the actual position of an embodiment of a guide body according to the present disclosure that is being placed on to a distal end of a femur.
FIG. 2B is an example of a fluoroscopic image taken in a sagittal plane (lateral view) that may be displayed to a user during a surgical procedure showing the divergence between the intended target position of the guide body and the actual position of an embodiment of the guide body shown in FIG. 2A.
FIG. 3 is an illustration showing a drill bit being used to drill the patient's bone using the guide assembly of the present disclosure.
FIGS. 4A-4D are illustrations showing the corner protectors used in combination with the guide assembly of the present disclosure.
FIG. 5 is a flowchart illustrating an example of a method according to the present disclosure.
FIG. 6 is a schematic illustration of a surgical system of the present disclosure.
FIG. 7A is an illustration of an operational jig according to another embodiment of the present disclosure that incorporates an optical fiducial.
FIG. 7B is an illustration showing a honeycomb raster example of the optical fiducial of FIG. 7A.
FIG. 8 is a schematic illustration of an optical imaging system of the present disclosure.
FIG. 9 is a flowchart illustrating an example of another method according to the present disclosure.

All illustrations shown in the figures are schematic and are not intended to show actual dimensions or proportions.

### DETAILED DESCRIPTION

This description of preferred embodiments is intended to be read in connection with the accompanying drawings, which are to be considered part of the entire written description of this invention. The drawing figures are not necessarily to scale and certain features of the invention may be shown exaggerated in scale or in somewhat schematic form in the interest of clarity and conciseness. In the description, relative terms such as "horizontal," "vertical," "up," "down," "top," and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

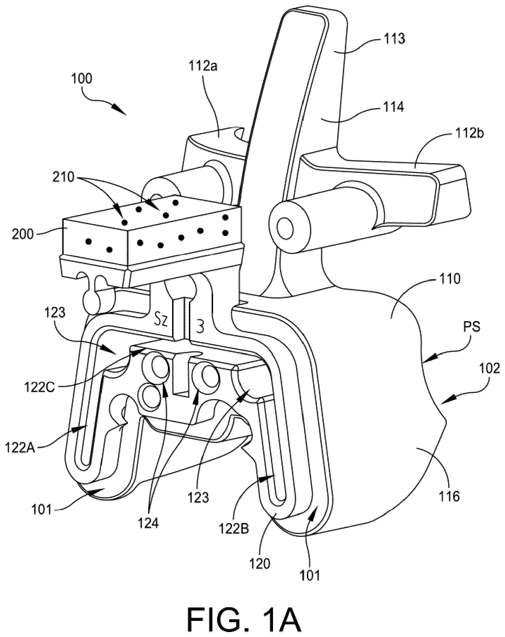

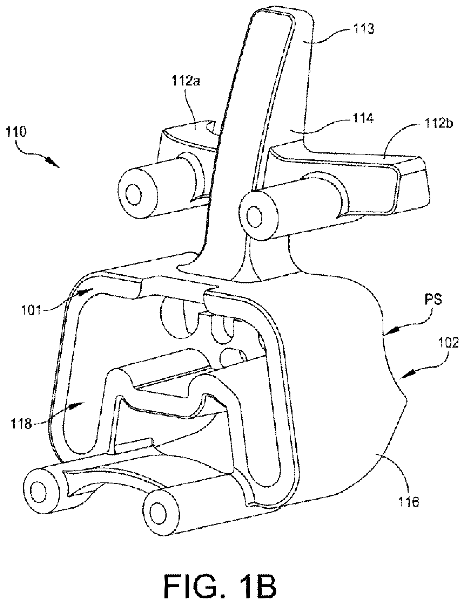

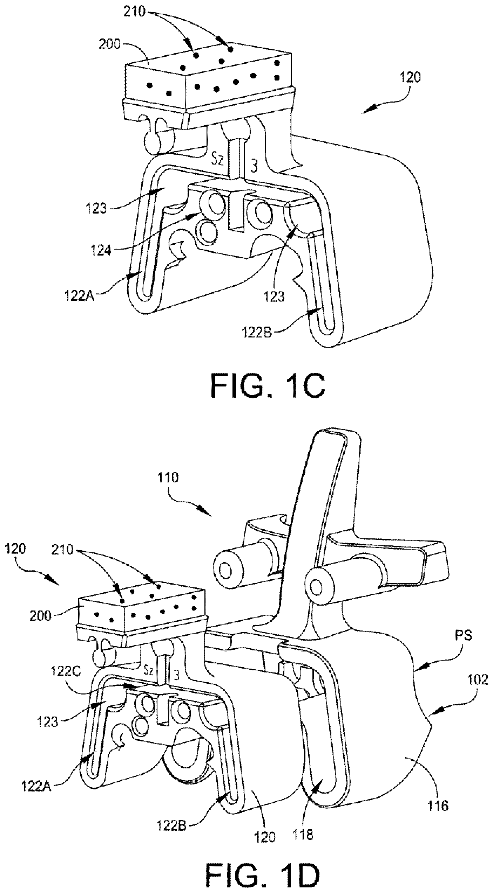

FIGS. 1A-1D is an illustration of a guide assembly **100** according to an embodiment. The guide assembly **100** is not a monolithic structure but includes at least two major components: a patient-matched portion **110** that is only useful for a particular patient, and a reusable portion **120** that is configured to be useable with many different patient-matched portions.

In the illustrated example shown, the patient-matched portion **110** is a patient-matched guide body **110** used in bone resection procedure, and the reusable portion **120** is an operational jig **120** that mates with the patient-matched guide body **110.** The guide assembly 100 can be configured as a tibia resection guide for use in a surgical procedure for resecting the distal end of a tibia in a total ankle arthroplasty. The patient-matched guide body **110** can be formed from a resilient polymer material of the type that can be made using an additive manufacturing process such as 3D printing.

The patient-matched guide body **110** can comprise a unitary block structure with bone engaging surface features configured for complementarily matching with anatomical surface features of a selected region of the patient's natural bone (e.g., a portion of the tibia).

For example, the patient-matched guide body **110** can include a cruciform tibia yoke **114** projecting upwardly from a base **116** of the guide body **110.** The patient-matched guide body **110** and the operational jig **120** are configured to be assembled together to form the guide assembly **100.** Thus the patient-matched guide body **110** includes a guide receptacle **118** for receiving at least a portion of the operational jig **120.**

The cruciform tibia yoke **114** can include a pair of spaced apart arms **112a, 112b** that project outwardly from a central post **113.** The base **116,** arms **112a, 112b,** and the central post **113** can each have a conformal bone engaging surface that can be configured for complementary matching of the anatomical surface features of a selected region of the patient's natural bone using patient specific instrument software using images of the patient. In the illustrated example, the conformal bone engaging surfaces are complementary to the contours of a corresponding portions of the distal end of the patient's tibia.

The operational jig **120** can be a cutting guide and/or a drill guide for guiding a cutting instrument or a drilling instrument during a bone resection procedure. For example, the operational jig **120** can include features for guiding a saw, drill, or other tools. The operational jig **120** can also include features particularly adapted to allow additional components to be connected to the patient-matched guide body **110,** as will be further described. The operational jig **120** can include features that enable connection to one or more of a variety of cutting guides, depending on the particular surgical procedure. The guide receptacle **118** and the operational jig **120** have complementary shape.

The patient-matched guide body **110** includes a first side **101** and a second side **102** opposite the first side. The second side **102** is the side that engages the patient bone and the second side comprises at least one patient-specific surface **PS** that is configured for complementarily engaging the anatomical surface features of a target area of the patient's natural bone. The patient-matched surfaces **PS** are configured with contoured surfaces that complementarily match the contours of a particular portion of the patient's bone surface that the guide body **110** is intended to engage. Thus, the patient-matched guide body **110** is only usable for a particular patient and it is a disposable component of the guide assembly **100.** In contrast, the operational jig **120** can be reused with a different patient. In such a case, the operational jig **120** would be used in combination with a different patent-matched guide body that is custom fabricated to match the anatomical bone surface features of that other patient. As such, the operational jig **120** is formed of a surgical grade durable material such as metal or ceramic.

To properly use the patient-matched surgical instruments such as the patient-matched guide body **110,** the patient-matched surfaces **PS** of the patient-matched guide body **110** needs to be accurately positioned and aligned onto the intended site on the patient's bone. Thus, ability to visually compare the position and alignment of the patient-matched guide body 110 against the intended position and alignment of the patient-matched guide body during the surgical procedure would be helpful.

The present disclosure describes new features for a patient-matched surgical instruments such as the illustrated patient-matched guide body **110** that enables such visualization of the position and alignment of the patient-matched surgical instrument and the related surgical system.

### [Locating with Radio-opaque markers]

Referring to FIGS. 1A-1D, the operational jig **120** can comprise a reference body **200** that contains a plurality of radio-opaque markers **210.** The radio-opaque markers **210** function as markers representing the guide assembly's orientation and position with respect to the patient body when the portion of the patient's body on which the guide assembly **100** is being placed is viewed with an X-ray imaging tool, such as fluoroscope. Thus, the radio-opaque markers **210** incorporated into the operational jig **120** can be used to check the alignment of the guide assembly **100** after the guide assembly is positioned on the patient's bone and before the bone resection is commenced. The alignment of the guide assembly **100** here is referring to the alignment of the guide assembly in the 3-dimensional space in reference to the patient's bone. In other words, the alignment encompasses the position, orientation, and attitude of the guide assembly **100.**

The radio-opaque markers **210** can be pieces of radio-opaque material and can be provided in the reference body **200** in a three-dimensional cluster of predefined pattern. Preferably, the pieces of radio-opaque material can have uniform shape and size. The radio-opaque material can be in the form of spheres of a desirable size.

The pattern of the cluster of the radio-opaque markers **210** can be asymmetric to readily indicate the orientation, position, and attitude of the reference body **200** when viewed with a fluoroscope. The reference body **200** is affixed to the guide assembly **100** at a predetermined location on the guide assembly **100.** Because the reference body **200** is affixed to the guide assembly **100** at a predetermined location, the relative positions of the various portions of the guide assembly **100** with respect to the reference body **200** is a defined parameter. Hence, when the location and orientation of the reference body **200** is known, the position and orientation of the guide assembly **100** can be determined. Thus, by locating the reference body **200** with a fluoroscope, the position and orientation of the guide assembly **100** can be determined.

The plurality of radio-opaque markers **210** are placed in the reference body **200** at known predetermined locations so that the exact placement of the radio-opaque markers **210** and the pattern formed by the radio-opaque markers **210** are known parameters. Because they are known parameters, when viewed with a fluoroscope, for example, the placement and orientation of the reference body **200** and, in turn, the guide assembly **100** can be readily ascertained.

The reference body **200** can be integrated into the structure of the guide assembly **100** in different ways. In some embodiments, the reference body **200** can be integrated into the structure of the patient-matched guide body **110.**

In some embodiments, the reference body **200** can be integrated into the structure of the operational jig **120.** Because the operational jig **120** is a reusable component that can be used with any patient-matched guide body **110** regardless of the differences in the patient-matched surface features of the patient-matched guide bodies, integrating the reference body **200** into the operational jig **120** can be more economical.

In some embodiments, the reference body **200** can be permanently integrated into the operational jig **120** so that the reference body **200** is not removable from the operational jib **120.** In some embodiments, the reference body **200** can be provided as a modular unit that can be removably attached to the operational jig **120.** The modular connection between the reference body **200** and the operational jig **120** can be achieved by any one of the known mechanical connection arrangements. For example, the two components can engage in sliding matter with a spring-loaded detent mechanism for securely holding the two components.

Whether the reference body **200** is integrally formed into the operational jig **120** or provided as a modular unit, having the reference body **200** be a part of the operational jig **120** rather than the guide body **110** can be cost effective because unlike the patient-matched guide body **110,** which is only useable for a specific patient, the operational jig **120** is reusable.

In some embodiments, the plurality of radio-opaque markers **210** can be incorporated into the patient-matched guide body **110** itself. The radio-opaque markers can be integrally formed into the patient-matched guide body **110** using additive manufacturing process. In such embodiment, the plurality of radio-opaque markers **210** are placed in the patient-matched guide body **110** at predetermined locations so that the exact placement of the radio-opaque markers **210** and the pattern thus formed by the cluster of radio-opaque markers **210** are known parameters.

Regardless of the particular way the radio-opaque markers **210** are incorporated with the guide assembly **100,** because the locations of the radio-opaque markers **210** and the orientation of the pattern formed by them in relation to the structures of the guide assembly **100** are known parameters, when the guide assembly **100** is viewed, along with the patient's body portion on which the guide assembly **100** is being placed, with X-ray imaging device such as a fluoroscope, the outline of the patient-matched guide body **110** can be extrapolated from the X-ray image of the radio-opaque markers **210.** A surgical system **600** disclosed herein can present an X-ray image of the patient's anatomy and overlay the outline of the patient-matched guide body **110** on a display along with an overlaid image of the target outline of the patient-matched guide body **110** where the target outline represents the intended position of the patient-matched guide body **110** so that a surgeon can visually assess the placement and orientation of the patient-matched guide body **110** relative to the intended placement and orientation and make appropriate adjustments.

### [Surgical system]

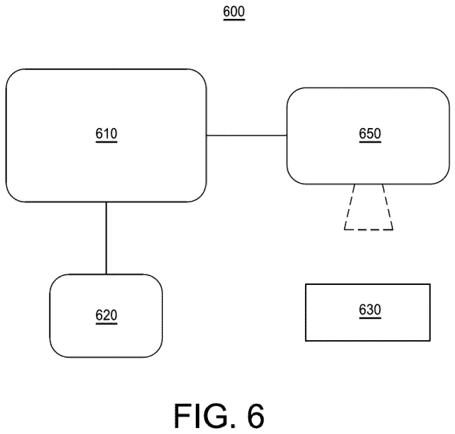

With either of the embodiments of the patient-matched guide assembly **100** disclosed herein, one where the radio-opaque markers **210** are provided in a separate reference body **200,** and a second one where the radio-opaque markers **210** are integrally incorporated within the guide body **110,** a surgical system **600** for providing a real-time feedback on the proper placement and alignment of the guide assembly **100** on the patient's bone can be configured and provided. A schematic representation of such surgical system **600** is shown in FIG. 6. The surgical system **600** can provide a feedback using an X-ray imaging device **650** such as a fluoroscope regarding the quality of the placement and alignment of the patient-matched guide assembly **100** after it is initially placed on the patient's bone. The system **600** can include a computing device **610** with a display screen **620,** a holder assembly **630** for holding the patient in such a manner that the part of the patient being operated on, the ankle for example, can be viewed under the X-ray imaging device **650.** The resulting X-ray image would be shown on the display screen **620** for the user (e.g. a surgeon).

In some embodiments, the computing device **610** can be configured with appropriate software to display the X-ray image (e.g. fluoroscopic image) of the part of the patient being operated on onto the display screen **620.**

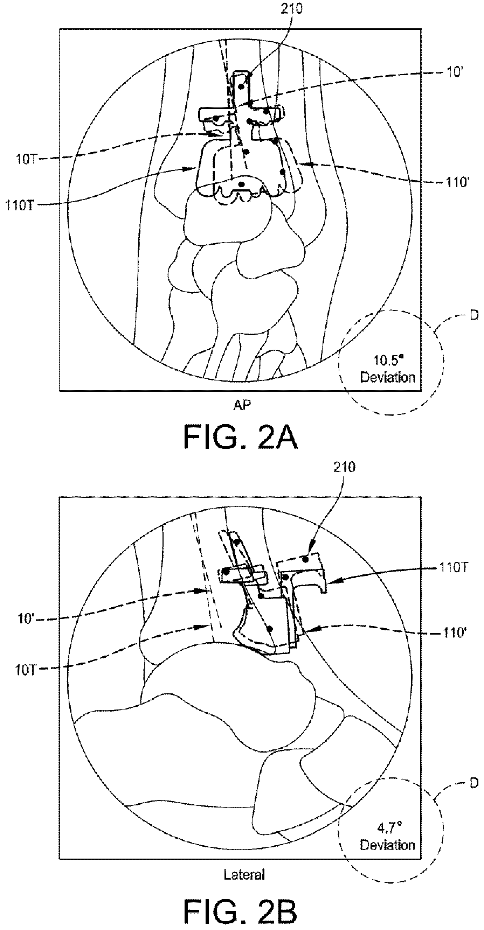

Examples of such X-ray image outputs are schematically illustrated in FIGS. 2A-2B. FIG. 2A is an frontal plane view (Anterior-Posterior (AP) view) fluoroscope image of the patient's ankle joint. The computing device **610** is configured to project and overlay onto the fluoroscopic image an outline or a line image of a pre-determined intended target position **110T** of the patient-matched guide body **110** and the associated target longitudinal axis **10T.**

Also shown in the fluoroscopic image in FIG. 2A is an outline or a line image of the actual patient-matched guide body **110** that is being placed on to the distal end of a femur. In this example, the patient-matched guide body **110** is that of the embodiment where the plurality of radio-opaque markers **210** are integrally formed with or embedded into the patient-matched guide body **110.** The radio-opaque markers **210** are visible as dark spots in the fluoroscopic image. FIG. 2B is a sagittal plane view (lateral view) fluoroscopic image of the same patient ankle with the same patient-matched guide body **110** seen in FIG. 2A.

Because the exact placement and the pattern of the plurality of radio-opaque markers **210** within the volume of the patient-matched guide body **110** is precisely known, the location of the outline of the patient-matched guide body **110** can be determined by extrapolating from the locations of the radio-opaque markers **210** in the fluoroscopic image. Thus, the computing device **610** of the surgical system **600** can extrapolate and project (*i*.*e*., display) an outline image **110'** of the patient-matched guide body **110** on the system's display screen **620** so that the surgeon can compare the actual location of the patient-matched guide body **110** represented by the outline image **110'** to the pre-determined target position **110T.**

Additionally, based on the outline **110'** of the patient-matched guide body **110,** the computing device **610** of the system **600** can define a longitudinal axis **10'** of the patient-matched guide body **110** and project and overlay an image of the longitudinal axis **10'** of the patient-matched guide body **110** (this is also the longitudinal axis of the guide assembly **100)** over the fluoroscopic image and display it on the system's display screen **620** for the user. To provide the user with a real-time feedback on the quality of the placement and alignment of patient-matched guide body **110,** the system **600** is configured to project an outline of the pre-determined intended target position **110T** of the patient-matched guide body **110** and the associated target longitudinal axis **10T** onto the live fluoroscopic image feed.

Furthermore, as shown in the example AP view in FIG. 2A and the example lateral view in FIG. 2B, based on the projections of the longitudinal axis **10'** and the target longitudinal axis **10T,** the system can calculate and display the angular deviation **D** in each of the views.

It should be noted that because the pattern of the radio-opaque markers **210,** and the geometry and structures of the operational jig **120** and the patient-matched guide body **110** are in 3D while the X-ray image produced by the X-ray imaging device **650** is in 2D, the computing device **610** is equipped with appropriate software that perform the data transformation from the 3D parameters to 2D projection images so that the outline of the patient-matched guide body **110,** which is a 3D structure, is presented on the display **620** as the outline image **110'** which is a 2D projection of the 3D structure of the patient-matched guide body **110.** The same applies to the 2D projection image of the outline image of the target position **110T** of the patient-matched guide body **110.**

The system for providing a real-time feedback on the proper placement and alignment of the guide assembly can work the same way with the embodiment of the patient-matched guide assembly **100** in which the radio-opaque markers **210** are provided in the reference body **200** that attaches to the operational jig **120.** Because the 3D cluster pattern of the radio-opaque markers **210** in the reference body **200** is a pre-determined one and, thus, the relative position of the patient-matched guide body **110** with respect to the 3D cluster pattern of the radio-opaque markers **210** is precisely known, from a fluoroscopic image, the system can extrapolate and project an outlined image of the patient-matched guide body **110** overlaid on the fluoroscopic image on the system's display screen. Outlined image of the pre-determined intended target position **110T** of the patient-matched guide body **110** can also be overlaid on the fluoroscopic image at the same time so that the surgeon can compare the actual position of the patient-matched guide body to the intended target position **110T.**

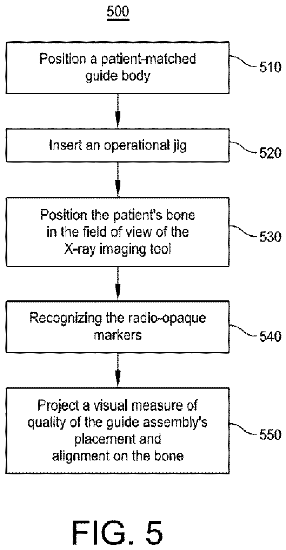

Referring to FIG. 5, a method for a surgical procedure on a bone of a patient according to an embodiment of the present disclosure is illustrated in the flowchart 500. The method comprises:
(a) positioning a patient-matched guide body **110** on the bone, where the patient-matched guide body **110** comprises a recess **118** configured to receive an operational jig **120** (block **510);**
(b) inserting the operational jig **120** into the recess **118** in the patient-matched guide body **110** thus forming a guide assembly **100** that comprises the patient-matched guide body **110** and the operational jig **120,** where the operational jig **120** or the patient-matched guide body **110** includes a plurality of radio-opaque markers **210** that represent the guide assembly's placement and alignment with respect to the bone when viewed with an X-ray imaging tool, (at block **520);**
(c) positioning the patient's bone where the patient-matched guide body **110** is placed in the field of view of the X-ray imaging tool (at block **530);**
(d) recognizing the radio-opaque markers **210** and extrapolating the outline of the patient-matched guide body **110** (at block **540);** and
(e) projecting, on the X-ray imaging tool's display, a visual measure of quality of the guide assembly's placement and alignment on the bone, (at block **550).** This step (e) involves the projection of an outline of the pre-determined intended target position **110T** of the patient-matched guide body **110** and the associated target longitudinal axis **10T** on the system's display screen as discussed above. The system can further project or display the angular deviation **D** between the pre-determined intended target longitudinal axis **10T** and the longitudinal axis **10'** of the guide body **110.** The longitudinal axis **10'** can also be the longitudinal axis of the whole guide assembly.

It should be noted that in some embodiments the guide body **110** and the operational jig **120** can be preassembled together before being placed on the patient. In such cases, the step (b) would happen first, then the step of positioning the assembled guide assembly 100 on the patient's bone would occur.

In some varied embodiments, a method for a surgical procedure on a bone of a patient can comprise:
(a') positioning a patient-matched guide body **110** on the bone, where the patient-matched guide body **110** comprises a recess **118** configured to receive an operational jig **120;**
(b') inserting the operational jig **120** into the recess **118** in the patient-matched guide body **110** thus forming a guide assembly **100** that comprises the patient-matched guide body **110** and the operational jig **120,** where the operational jig **120** includes a plurality of radio-opaque markers **210** that are markers representing the guide assembly's placement and alignment with respect to the bone when viewed with an X-ray imaging tool;
(c') positioning the patient's bone where the patient-matched guide body **110** is placed in the field of the view of the X-ray imaging tool;
(d') recognizing the radio-opaque markers **210** and extrapolating the outline of the patient-matched guide body **110;** and
(e') checking the orientation and position of the guide assembly **100** by viewing the plurality of radio-opaque markers **210** with an X-ray imaging tool;
(f') intraoperatively adjusting the orientation and position of the guide assembly **100** as necessary; and
(g') repeating the steps (c') - (f') as necessary.

In some embodiments of the method, the X-ray imaging tool can be a fluoroscope.

In some embodiments, the patient-matched guide body **110** can comprise at least one patient-specific surface **PS** and the steps (a) and (a') can involves placing the at least one patient-specific surface **PS** into contact with the bone.

In some embodiments, the operational jig **120** is a resection guide comprising one or more guiding slots **122A, 122B, 122C** for bone cutting blades and the method further comprises a step of resecting the bone guided by the operational jig **120.**

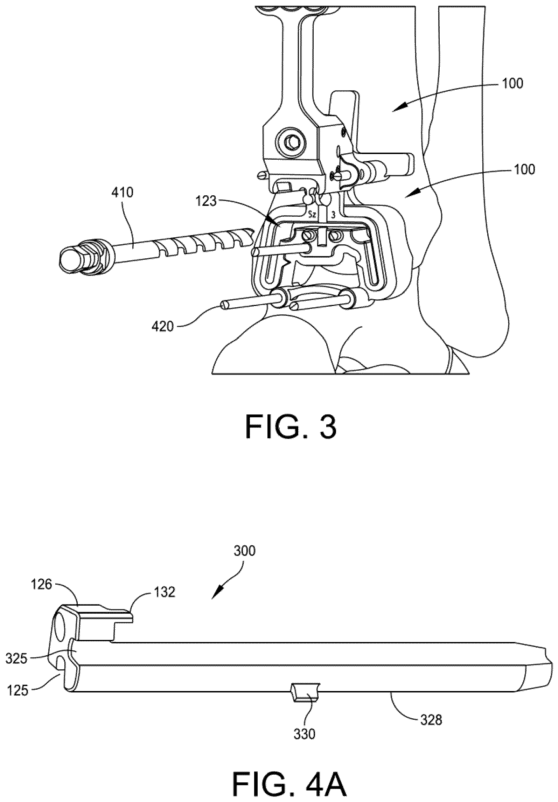

In some embodiments, the operational jig **120** is a drill guide comprising one or more drill guiding holes **123** for drill bits and the method further comprises a step of drilling the bone guided by the operational jig **120.** FIG. 3 shows a guide assembly **100** in place on the patient's tibia whose operational jig **120** includes such drill guiding holes **123** where each drill guiding hole **123** is configured for receiving and guiding a drill bit **410.**

In some embodiments, the operational jig **120** is a pin guide comprising one or more pin guiding holes **124** for surgical pins and the method further comprises a step of driving a surgical pin into the bone guided by the operational jig **120.** FIG. 3 shows a guide assembly **100** in place on the patient's tibia whose operational jig **120** includes such pin guiding holes **124** where each pin guiding hole **124** is configured for receiving and guiding a pin **420.**

In some embodiments, the operational jig **120** can be configured such that the drill guiding holes **123** and/or the pin guiding holes **124** are provided on a gimbal-like component that can adjust the orientation of the drill guiding holes **123** and/or the pin guiding holes **124** without adjusting the orientation and position of the whole guide assembly **100** on the patient's bone. Such gimbal-like component on an operational jig or a drill guide is disclosed in U.S. patent Application Serial No. 18/312,648, filed on May 5, 2023, the entire contents of which are incorporated herein by reference. The benefit of this embodiment is that when the placement and orientation of the guide assembly **100** deviates from the originally intended and planned position on the bone, the adjustability of the drill guiding holes **123** and/or the pin guiding holes **124** by operation of the gimbal-like component can compensate for that deviation. In such embodiments, the step (d') would involve intraoperatively adjusting the orientation and position of the operational jig's drill guiding holes and/or pin guiding holes as necessary.

In some embodiments, the bone is a distal end of a tibia in an ankle joint. In some embodiments, the operational jig **120** is a resection guide comprising one or more guiding slots **122A, 122B, 122C** for bone cutting blades and the method further comprises a step of resecting the distal end of the tibia guided by the operational jig **120** as part of an ankle arthroplasty.

In some embodiments, the operational jig **120** is a drill guide comprising one or more guiding holes **123** for drill bits and the method further comprises a step of drilling the distal end of the tibia guided by the operational jig **120** as part of an ankle arthroplasty.

In some embodiments, the operational jig **120** is a pin guide comprising one or more guiding holes **124** for surgical pins and the method further comprises a step of driving a surgical pin into the distal end of the tibia guided by the operational jig **120** as part of an ankle arthroplasty.

In some embodiments, the bone is a distal end of a femur in a knee joint. In some embodiments, the operational jig **120** is a resection guide comprising guiding slots **122** for bone cutting blades and the method further comprises a step of resecting the distal end of the femur guided by the operational jig **120** as part of a knee arthroplasty.

In some embodiments, the operational jig **120** is a drill guide comprising one or more guiding holes **123** for drill bits and the method further comprises a step of drilling the distal end of the femur guided by the operational jig **120** as part of a knee arthroplasty.

In some embodiments, the operational jig **120** is a pin guide comprising one or more guiding holes **124** for surgical pins and the method further comprises a step of driving a surgical pin into the distal end of the femur guided by the operational jig **120** as part of a knee arthroplasty.

In some embodiments, the bone is a proximal end of a tibia in a knee joint. In some embodiments, the operational jig **120** is a resection guide comprising one or more guiding slots **122A, 122B, 122C** for bone cutting blades and the method further comprises a step of resecting the proximal end of the tibia guided by the operational jig **120** as part of a knee arthroplasty.

In some embodiments, the operational jig **120** is a drill guide comprising one or more guiding holes **123** for drill bits and the method further comprises a step of drilling the proximal end of the tibia guided by the operational jig **120** as part of a knee arthroplasty.

In some embodiments, the operational jig **120** is a pin guide comprising one or more guiding holes **124** for surgical pins and the method further comprises a step of driving a surgical pin into the proximal end of the tibia guided by the operational jig **120** as part of a knee arthroplasty.

The step (a) and (a') of positioning the patient-matched guide body **110** on the bone can involve alignment and securing steps involving the use of pins and/or wires. Such procedures are described for example in U.S. publication No. 2022/0370081, the entire disclosure of which is incorporated herein by reference. After the guide assembly **100** is secured to the patient bone, a surgical procedure performed using the aid of the guide assembly **100** may include cutting and/or drilling to perform resection cuts. For instance, the surgeon may drill into the guiding holes **123,** as shown in FIG. 3 and subsequently perform resection cuts following the guide slots **122.**

Referring to FIG. 1C, in the context of the guide assembly **100** that is configured with three cutting guide slots **122A, 122B, 122C** for cutting a three-sided box cut at the distal end of a tibia, the two guide holes **123** in the operational jig **120** are positioned at two corners. First corner is defined between the cutting guide slot **122A** and **122C.** The second corner is defined between the cutting guide slot **122B** and **122C.**

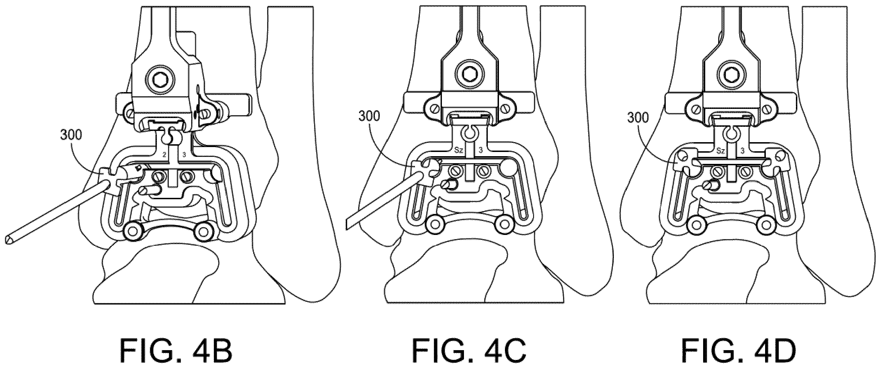

Referring to FIGS. 4A-4D, the disclosed guide assembly **100** may further include corner protectors **300.** After the guide assembly **100** is positioned on the patient's bone and holes are drilled into the bone guided by the two drill guide holes **123,** a corner protector **300** can be inserted into each of the holes drilled in the bone as illustrated in FIGS. 4B-4D. The corner protectors **300** are configured to be connected to the operational jig **120.** The corner protectors **300** work as stops and help limit the resection cuts made using the cutting guide slots **122A, 122B,** and **122** from extending into the drilled-out holes, i.e., the corner regions between the two adjacent cuts. More detailed description of such corner protectors **300** can be found for example in U.S. publication No. 2022/0370081.

In some embodiments, the operational jig **120** is coupled to the patient-matched guide body **110** and secured by a corner protector **300** configured to retain a spaced apart relation between the guide assembly and aligning guide holes for placement of a surgical pin into the bone guided by the guide assembly. In the current product we use "corner protectors" as seen below.

In some embodiments, the method can further comprise fabricating the patient-matched guide body before step (a) and (a').

Also disclosed is a surgical kit according to another embodiment. The kit comprises a guide assembly **100** for attaching to a patient's bone to guide bone resection procedures. The guide assembly includes: a patient-matched guide body **110;** an operational jig **120;** and one or more corner protectors **300.** The operational jig **120** comprises: a plurality of radio-opaque markers **210;** one or more cutting guide slots **122A, 122B, 122C;** and one or more drill guide holes **124,** wherein the radio-opaque markers representing the guide assembly's orientation and position with respect to the bone when viewed with an X-ray imaging tool. The patient-matched guide body **110** comprises: a first side **101;** a second side **102** opposite the first side; and a guide receptacle **118** for receiving the operational jig **120,** where the second side includes at least one patient-specific surface **PS** that is configured for complementarily engaging the anatomical surface features of a target area of a patient's bone.

### [Locating with Optical Fiducials]

Also disclosed is an embodiment of a guide assembly **100** that incorporates one or more optical fiducials that can be used with an optical imaging system to determine the position and orientation of the guide assembly **100** in an optical image, extrapolate the outline of the guide assembly **100** and overlay the outline onto the optical image along with an overlaid target outline of the guide assembly where the target outline represents the intended target position and orientation of the guide assembly **100.**

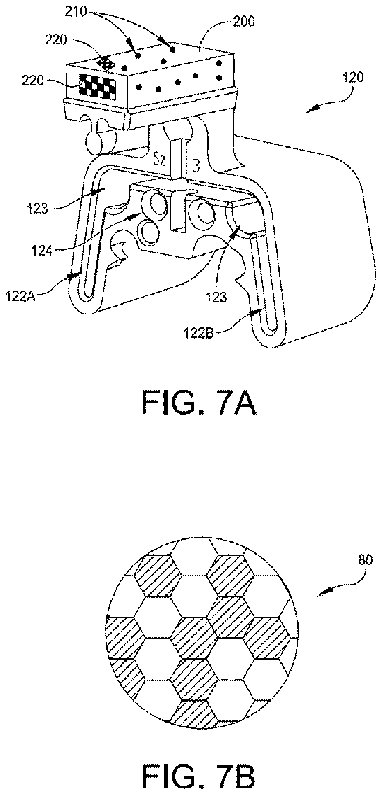

FIG. 7A is an illustration of an operational jig **120** according to an embodiment of the present disclosure in which the reference body **200** includes an optical fiducial **220** that can be used by an optical imaging system to determine the position and orientation of the guide assembly **100.** The optical fiducial **220** has an optical pattern **80** thereon that is a unique pattern that can be recognized by the optical imaging system to determine the location and orientation of the structure, in this example the operational jig **120,** on which the optical fiducial **220** is provided. The optical pattern **80** can be printed directly to the reference body **200.** It should be noted that in some embodiments the optical fiducial **220** can be provided on the patient-matched guide body **110** rather than on the operational jig **120.** Optical fiducial **220** may include coded digital information, similar to QR (Quick Response) codes, which identifies the 3D printed guide body **110** that corresponds with the patient matched case number. This digital information can be used during surgery to confirm that correct components are assembled for that specific surgery and side of the anatomy. This may be particularly useful where multiple guide bodies **110** are to be used in the same surgery, such as simultaneous arthroplasty on both left and right sides.

The optical pattern **80** can be composed of a geometrically even raster of light and dark fields. The light and dark fields can be square or round fields or have a shape that has a certain fit to a rectangular raster. The color of the raster fields can be any differing color, including printing dark or black fields onto a light colored or metallic surface. Instead of light and dark fields, fields of different color may be used, e.g. red and green, yellow and blue, or yellow and black. As an alternative, the optical pattern **80** can be composed of a honeycomb raster of light and dark fields as shown in the example illustrated in FIG. 7B. The fields may be hexagonal or round fields, or have a shape which has a certain fit to a honeycomb raster. The color of the honeycomb raster fields can be any differing color, including printing dark or black fields onto a light colored or metallic surface.

The optical fiducial **220** is fixedly mounted to the reference body **200** so that the relative spatial relationship between the geometry and structure of the reference body **200** and the optical pattern **80** on the optical fiducial **220** is a known parameter. In turn, the relative spatial relationship between the optical pattern **80** on the optical fiducial **220** and the geometry and structure of the operational jig **120** and the patient-matched guide body **110** is a known parameter because the spatial relationship between the reference body **200** and the operational jig **120** and the patient-matched guide body **110** is a fixed and known parameter.

Because the location of the optical fiducial **220** and the orientation of the optical pattern **80** thereon in relation to the structures of the components of the guide assembly **100** are known parameters, when the guide assembly **100** is viewed, along with the patient's body portion on which the guide assembly **100** is being placed, with an optical imaging device (*i.e*., an optical digital camera) the outline of the patient-matched guide body **110** can be extrapolated from the optical image of the optical pattern **80.**

Also disclosed is an optical system **700** for providing a real-time feedback on the proper placement and alignment of the guide assembly **100** on the patient's bone using the optical fiducial **220.** The optical system **700** presents an optical digital image of the patient's anatomy and overlay the outline of the patient-matched guide body **110** on a display along with an overlaid image of the target outline of the patient-matched guide body **110** where the target outline represents the intended position of the patient-matched guide body **110** so that a surgeon can visually assess the placement and orientation of the patient-matched guide body **110** relative to the intended placement and orientation and make appropriate adjustments.

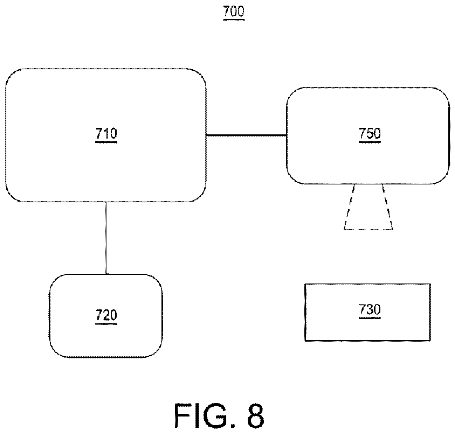

A schematic representation of such optical system **700** is shown in FIG. 8. The optical system **700** can include a computing device **710** with a display screen **720,** a holder assembly **730** for holding the patient in such a manner that the part of the patient being operated on, the ankle for example, can be viewed under the optical imaging device **750** such as an optical digital camera. The resulting image would be shown on the display screen **720** for the user (e.g. a surgeon).

In some embodiments, the computing device **710** can be configured with appropriate software to display the image of the part of the patient being operated on onto the display screen **720.** The resulting image would be similar to the X-ray images shown in FIGs. 2A and 2B except that instead of X-ray images of the patient's anatomy being shown, a digital picture output from the digital camera **750** would be shown on the display screen **720.** Similar to the X-ray examples shown in FIGs. 2A and 2B, the digital pictures from the digital camera **750** would be displayed with the extrapolated outline image **110'** of the patient-matched guide body **110** and the outline image of the intended target position **110T** of the patient-matched guide body **110** would be overlaid in the digital picture. The longitudinal axis **10'** of the patient-matched guide body **110** and the longitudinal axis **10T** for the intended target position **110T** would also be overlaid in the digital picture.

As noted above in the context of the embodiments in which the guide assembly **100** was being located using X-ray images of the radio-opaque markers, it should be noted similarly here that because the pattern of the optical pattern **80** on the optical fiducial **220,** and the geometry and structures of the operational jig **120** and the patient-matched guide body **110** are in 3D while the image produced by the optical imaging device **750** is in 2D, the computing device **710** is equipped with appropriate software that perform the data transformation from the 3D parameters to 2D projection images so that the outline of the patient-matched guide body **110,** which is a 3D structure, is presented on the display **720** as an outline image **110'** which is a 2D projection of the 3D structure of the patient-matched guide body **110.** The same applies to the 2D projection image of the outline image of the target position **110T** of the patient-matched guide body **110.**

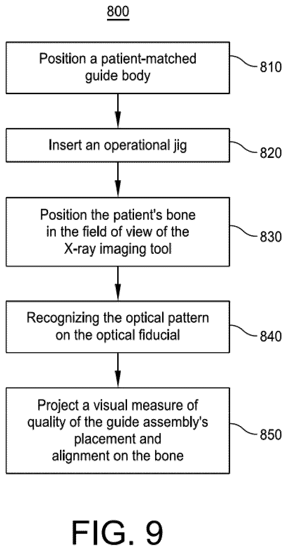

Referring to FIG. 9, a method for a surgical procedure on a bone of a patient according to an embodiment of the present disclosure is illustrated in the flowchart **800.** The method comprises:
(a) positioning a patient-matched guide body **110** on the bone, where the patient-matched guide body **110** comprises a recess **118** configured to receive an operational jig **120** (block **810);**
(b) inserting the operational jig **120** into the recess **118** in the patient-matched guide body **110** thus forming a guide assembly **100** that comprises the patient-matched guide body **110** and the operational jig **120,** where the operational jig **120** or the patient-matched guide body **110** includes one or more optical fiducial **220** that represent the guide assembly's placement and alignment with respect to the bone when viewed with an optical imaging tool, where the optical fiducial **220** includes an optical pattern **80,** (at block **820);**
(c) positioning the patient's bone where the patient-matched guide body **110** is placed in the field of view of the optical imaging tool **750** and acquiring a 2D digital image (at block **830);**
(d) recognizing the optical pattern on the optical fiducial **220** and extrapolating the outline of the patient-matched guide body **110** (at block **840);** and
(e) projecting, on the surgical system's display, a visual measure of quality of the guide assembly's placement and alignment on the bone, (at block **850).** This step (e) involves the projection of an outline of the pre-determined intended target position **110T** of the patient-matched guide body **110** and the associated target longitudinal axis **10T** on the system's display screen as discussed above. The system can further project or display the angular deviation **D** between the pre-determined intended target longitudinal axis **10T** and the longitudinal axis **10'** of the guide body **110.** The longitudinal axis **10'** can also be the longitudinal axis of the whole guide assembly.

It should be noted that in some embodiments the guide body **110** and the operational jig **120** can be preassembled together before being placed on the patient. In such cases, the step (b) would happen first, then the step of positioning the assembled guide assembly **100** on the patient's bone would occur.

In some embodiments, both the radio-opaque markers **210** and the optical fiducial **220** features may be incorporated into the guide assembly **100.** In which case, the system **600** and **700** can be consolidated appropriately to reduce the number of system components. For example, the functions of the computing device **610** and **710** can be accomplished by one computing device. The display screen **620** and **720** can be consolidated into one display screen. The holder assemblies **630** and **730** for holding the patient can be consolidated into one assembly.

### [List of non-limiting illustrative embodiments]

The following is a list of non-limiting illustrative embodiments disclosed herein:
Illustrative embodiment 1. A method for a surgical procedure on a bone of a patient, the method comprising:
   (a) positioning a patient-matched guide body on the bone, wherein the patient-matched guide body comprises a recess configured to receive an operational jig;
   (b) inserting the operational jig into the recess in the patient-matched guide body thus forming a guide assembly that comprises the patient-matched guide body and the operational jig, wherein the operational jig or the patient-matched guide body includes a plurality of radio-opaque markers that represent the guide assembly's placement and alignment with respect to the bone when viewed with an X-ray imaging tool;
   (c) positioning the patient's bone where the patient-matched guide body is placed in the field of view of the X-ray imaging tool;
   (d) recognizing the radio-opaque markers and extrapolating the outline of the patient-matched guide body; and
   (e) projecting, on the X-ray imaging tool's display, a visual measure of quality of the guide assembly's placement and alignment on the bone.
Illustrative embodiment 2. The method of illustrative embodiment 1, wherein the step (e) comprises projecting an outline of the patient-matched guide body and an outline of pre-determined intended target position of the patient-matched guide body.
Illustrative embodiment 3. The method any one of illustrative embodiments 1-2, wherein the step (e) further comprises projecting a longitudinal axis of the patient-matched guide body and target longitudinal axis of the pre-determined intended target position of the patient-matched guide body.
Illustrative embodiment 4. The method of illustrative embodiment 3, further comprising (f) projecting, on the X-ray imaging tool's display, an angular deviation between the longitudinal axis of the patient-matched guide body and the target longitudinal axis of the pre-determined intended target position of the patient-matched guide body.
Illustrative embodiment 5. The method of any one of illustrative embodiments 1-4, wherein the X-ray imaging tool is a fluoroscope.
Illustrative embodiment 6. The method of any one of illustrative embodiments 1-5, wherein the operational jig comprises the plurality of radio-opaque markers.
Illustrative embodiment 7. The method of illustrative embodiment 6, wherein the plurality of radio-opaque markers are integrally incorporated into a portion of the operational jig.
Illustrative embodiment 8. The method of illustrative embodiment 6, wherein the plurality of radio-opaque markers are provided in an aligner component that is attachable to the operational jig.
Illustrative embodiment 9. The method of illustrative embodiment 1, wherein the plurality of radio-opaque markers are integrally incorporated into the patient-matched guide body.
Illustrative embodiment 10. The method of illustrative embodiments 1-9, wherein the patient-matched guide body comprises at least one patient-specific surface and the step (a) involves placing the at least one patient-specific surface into contact with the bone.
Illustrative embodiment 11. The method of illustrative embodiments 1-10, wherein the operational jig is a resection guide comprising one or more guiding slots for bone cutting blades and the method further comprises a step of resecting the bone guided by the operational jig.
Illustrative embodiment 12. The method of illustrative embodiments 1-10, wherein the operational jig is a drill guide comprising one or more guiding holes for drill bits and the method further comprises a step of drilling the bone guided by the operational jig.
Illustrative embodiment 13. The method of illustrative embodiments 1-10, wherein the operational jig is a pin guide comprising one or more guiding holes for surgical pins and the method further comprises a step of driving a surgical pin into the bone guided by the operational jig.
Illustrative embodiment 14. The method of illustrative embodiments 1-13, wherein the bone is a distal end of a tibia in an ankle joint.
Illustrative embodiment 15. The method of illustrative embodiment 14, wherein the operational jig is a resection guide comprising one or more guiding slots for bone cutting blades and the method further comprises a step of resecting the distal end of the tibia guided by the operational jig as part of an ankle arthroplasty.
Illustrative embodiment 16. The method of illustrative embodiment 14, wherein the operational jig is a drill guide comprising one or more guiding holes for drill bits and the method further comprises a step of drilling the distal end of the tibia guided by the operational jig as part of an ankle arthroplasty.
Illustrative embodiment 17. The method of illustrative embodiment 14, wherein the operational jig is a pin guide comprising one or more guiding holes for surgical pins and the method further comprises a step of driving a surgical pin into the distal end of the tibia guided by the operational jig as part of an ankle arthroplasty.
Illustrative embodiment 18. The method of illustrative embodiments 1-13, wherein the bone is a distal end of a femur in a knee joint.
Illustrative embodiment 19. The method of illustrative embodiment 18, wherein the operational jig is a resection guide comprising guiding slots for bone cutting blades and the method further comprises a step of resecting the distal end of the femur guided by the operational jig as part of a knee arthroplasty.
Illustrative embodiment 20. The method of illustrative embodiment 18, wherein the operational jig is a drill guide comprising one or more guiding holes for drill bits and the method further comprises a step of drilling the distal end of the femur guided by the operational jig as part of a knee arthroplasty.
Illustrative embodiment 21. The method of illustrative embodiment 18, wherein the operational jig is a pin guide comprising one or more guiding holes for surgical pins and the method further comprises a step of driving a surgical pin into the distal end of the femur guided by the operational jig as part of a knee arthroplasty.
Illustrative embodiment 22. The method of illustrative embodiments 1-13, wherein the bone is a proximal end of a tibia in a knee joint.
Illustrative embodiment 23. The method of illustrative embodiment 22, wherein the operational jig is a resection guide comprising one or more guiding slots for bone cutting blades and the method further comprises a step of resecting the proximal end of the tibia guided by the operational jig as part of a knee arthroplasty.
Illustrative embodiment 24. The method of illustrative embodiment 22, wherein the operational jig is a drill guide comprising one or more guiding holes for drill bits and the method further comprises a step of drilling the proximal end of the tibia guided by the operational jig as part of a knee arthroplasty.
Illustrative embodiment 25. The method of illustrative embodiment 22, wherein the operational jig is a pin guide comprising one or more guiding holes for surgical pins and the method further comprises a step of driving a surgical pin into the proximal end of the tibia guided by the operational jig as part of a knee arthroplasty.
Illustrative embodiment 26. The method of illustrative embodiments 1-25, wherein the operational jig is coupled to the patient-matched guide body and secured by a corner protector configured to retain a spaced apart relation between the guide assembly and aligning guide holes for placement of a surgical pin into the bone guided by the guide assembly.
Illustrative embodiment 27. The method of illustrative embodiments 1-26, further comprising fabricating the patient-matched guide body before step (a).
Illustrative embodiment 28. A surgical system comprising:
   an X-ray imaging device;
   a display screen; and
   a computing device configured to display an X-ray image of a bone of a patient on which a patient-matched guide assembly is positioned, on the display screen;
      wherein the patient-matched guide assembly includes a plurality of radio-opaque markers representing the patient-matched guide assembly's placement and alignment with respect to the bone when viewed with the X-ray imaging device;
      wherein the computing device is further configured to project, on the display screen, a visual measure of quality of the patient-matched guide assembly's placement and alignment on the bone.
Illustrative embodiment 29. The surgical system of illustrative embodiment 28, wherein the patient-matched guide assembly comprises:
   a patient-matched guide body with at least one patient-matched bone contacting surface;
   wherein the computing device's projection of the visual measure of quality of the patient-matched guide assembly's placement and alignment on the bone comprises:
      projecting an outline of the patient-matched guide body and an outline of pre-determined intended target position of the patient-matched guide body, over the X-ray image of the bone on the display screen.
Illustrative embodiment 30. The surgical system of any of the illustrative embodiments 28-29, wherein the computing device's projection of the visual measure of quality of the patient-matched guide assembly's placement and alignment on the bone further comprises:
   projecting a longitudinal axis of the patient-matched guide body and target longitudinal axis of the pre-determined intended target position of the patient-matched guide body, over the X-ray image of the bone on the display screen.
Illustrative embodiment 31. The surgical system of any of the illustrative embodiments 28-30, wherein the computing device's projection of the visual measure of quality of the patient-matched guide assembly's placement and alignment on the bone further comprises:
   projecting an angular deviation between the longitudinal axis of the patient-matched guide body and the target longitudinal axis of the pre-determined intended target position of the patient-matched guide body, over the X-ray image of the bone on the display screen.
Illustrative embodiment 32. A kit comprising:
   a guide assembly for attaching to a patient's bone to guide bone resection procedures, the guide assembly comprises:
   an operational jig that comprises: a plurality of radio-opaque markers; one or more cutting guide slots; and one or more drill guide holes, wherein the radio-opaque markers representing the guide assembly's orientation and position with respect to the bone when viewed with an X-ray imaging tool; and
   a patient-matched guide body that comprises: a first side; a second side opposite the first side; and a guide receptacle for receiving the operational jig, wherein the second side includes at least one patient-specific surface that is configured for complementarily engaging the anatomical surface features of a target area of a patient's bone.
Illustrative embodiment 33. The kit of illustrative embodiment 32, further comprising one or more corner protectors.

Although the devices, kits, systems, and methods have been described in terms of exemplary embodiments, they are not limited thereto. Rather, the appended claims should be construed broadly, to include other variants and embodiments of the devices, kits, systems, and methods, which may be made by those skilled in the art without departing from the scope and range of equivalents of the claimed devices, kits, systems, and methods.

Further disclosed herein is the subject-matter of the following clauses:
1. A method for a surgical procedure on a bone of a patient, the method comprising:
   (a) positioning a patient-matched guide body on the bone, wherein the patient-matched guide body comprises a recess configured to receive an operational jig;
   (b) inserting the operational jig into the recess in the patient-matched guide body thus forming a guide assembly that comprises the patient-matched guide body and the operational jig, wherein the operational jig or the patient-matched guide body includes a plurality of radio-opaque markers that represent the guide assembly's placement and alignment with respect to the bone when viewed with an X-ray imaging device of a surgical system;
   (c) positioning the patient's bone where the patient-matched guide body is placed in field of view of the X-ray imaging device;
   (d) recognizing the radio-opaque markers and extrapolating outline of the patient-matched guide body; and
   (e) projecting, on the surgical system's display screen, a visual measure of quality of the guide assembly's placement and alignment on the bone.
2. The method of clause 1, wherein the step (e) comprises projecting an outline of the patient-matched guide body and an outline of pre-determined intended target position of the patient-matched guide body.
3. The method of clause 2, wherein the step (e) further comprises projecting a longitudinal axis of the patient-matched guide body and target longitudinal axis of the pre-determined intended target position of the patient-matched guide body.
4. The method of clause 3, further comprising (f) projecting, on the surgical system's display screen, an angular deviation between the longitudinal axis of the patient-matched guide body and the target longitudinal axis of the pre-determined intended target position of the patient-matched guide body.
5. The method of clause 1, wherein the X-ray imaging device is a fluoroscope.
6. The method of clause 1, wherein the operational jig comprises the plurality of radio-opaque markers.
7. The method of clause 6, wherein the plurality of radio-opaque markers are integrally incorporated into a portion of the operational jig.
8. The method of clause 6, wherein the plurality of radio-opaque markers are provided in an aligner component that is attachable to the operational jig.
9. The method of clause 1, wherein the plurality of radio-opaque markers are integrally incorporated into the patient-matched guide body.
10. The method of clause 1, wherein the patient-matched guide body comprises at least one patient-specific surface and the step (a) involves placing the at least one patient-specific surface into contact with the bone.
11. The method of clause 1, wherein the operational jig is a resection guide comprising one or more guiding slots for bone cutting blades and the method further comprises a step of resecting the bone guided by the operational jig.
12. The method of clause 1, wherein the operational jig is a drill guide comprising one or more guiding holes for drill bits and the method further comprises a step of drilling the bone guided by the operational jig.
13. The method of clause 1, wherein the operational jig is a pin guide comprising one or more guiding holes for surgical pins and the method further comprises a step of driving a surgical pin into the bone guided by the operational jig.
14. The method of clause 1, wherein the bone is a distal end of a tibia in an ankle joint and the operational jig is a resection guide comprising one or more guiding slots for bone cutting blades and the method further comprises a step of resecting the distal end of the tibia guided by the operational jig as part of an ankle arthroplasty.
15. The method of clause 1, wherein the bone is a distal end of a tibia in an ankle joint and the operational jig is a drill guide comprising one or more guiding holes for drill bits and the method further comprises a step of drilling the distal end of the tibia guided by the operational jig as part of an ankle arthroplasty.
16. The method of clause 1, wherein the bone is a distal end of a tibia in an ankle joint and the operational jig is a pin guide comprising one or more guiding holes for surgical pins and the method further comprises a step of driving a surgical pin into the distal end of the tibia guided by the operational jig as part of an ankle arthroplasty.
17. The method of clause 1, wherein the bone is a distal end of a femur in a knee joint.
18. The method of clause 17, wherein the operational jig is a resection guide comprising guiding slots for bone cutting blades and the method further comprises a step of resecting the distal end of the femur guided by the operational jig as part of a knee arthroplasty.
19. The method of clause 17, wherein the operational jig is a drill guide comprising one or more guiding holes for drill bits and the method further comprises a step of drilling the distal end of the femur guided by the operational jig as part of a knee arthroplasty.
20. The method of clause 17, wherein the operational jig is a pin guide comprising one or more guiding holes for surgical pins and the method further comprises a step of driving a surgical pin into the distal end of the femur guided by the operational jig as part of a knee arthroplasty.
21. A surgical system comprising:
   an X-ray imaging device;
   a display screen; and
   a computing device configured to display an X-ray image of a bone of a patient on which a patient-matched guide assembly is positioned, on the display screen;
   wherein the patient-matched guide assembly includes a plurality of radio-opaque markers representing the patient-matched guide assembly's placement and alignment with respect to the bone when viewed with the X-ray imaging device;
   wherein the computing device is further configured to project, on the display screen, a visual measure of quality of the patient-matched guide assembly's placement and alignment on the bone.
22. The surgical system of clause 21, wherein the patient-matched guide assembly comprises:
   a patient-matched guide body with at least one patient-matched bone contacting surface;
   wherein the computing device's projection of the visual measure of quality of the patient-matched guide assembly's placement and alignment on the bone comprises:
      projecting an outline of the patient-matched guide body and an outline of pre-determined intended target position of the patient-matched guide body, over the X-ray image of the bone on the display screen.
23. The surgical system of clause 21 or of any of clauses 21-22, wherein the computing device's projection of the visual measure of quality of the patient-matched guide assembly's placement and alignment on the bone further comprises:
   projecting a longitudinal axis of the patient-matched guide body and target longitudinal axis of the pre-determined intended target position of the patient-matched guide body, over the X-ray image of the bone on the display screen.
24. The surgical system of clause 21 or of any of clauses 21-23 wherein the computing device's projection of the visual measure of quality of the patient-matched guide assembly's placement and alignment on the bone further comprises:
   projecting an angular deviation between the longitudinal axis of the patient-matched guide body and the target longitudinal axis of the pre-determined intended target position of the patient-matched guide body, over the X-ray image of the bone on the display screen.
25. The surgical system of clause 22, wherein the patient-matched guide body includes a plurality of radio-opaque markers to represent the guide assembly's placement and alignment with respect to a bone when viewed with an X-ray imaging device.
26. The surgical system of clause 25, wherein the plurality of radio-opaque markers are integrally incorporated into a portion of an operational jig.
27. The surgical system of clause 26, wherein the plurality of radio-opaque markers are provided in an aligner component that is attachable to the operational jig.
28. The surgical system of clause 25, wherein the plurality of radio-opaque markers are integrally incorporated into the patient-matched guide body.
29. The surgical system of clause 22, wherein the patient-matched guide body comprises at least one patient-specific surface.
30. The surgical system of clause 26, wherein the operational jig is a resection guide comprising one or more guiding slots for bone cutting blades and the method further comprises a step of resecting the bone guided by the operational jig.
31. The surgical system of any of clauses 26 and 30, wherein the operational jig is a drill guide comprising one or more guiding holes for drill bits and the method further comprises a step of drilling the bone guided by the operational jig.
32. The surgical system of any of clauses 26, 30 and 31, wherein the operational jig is a pin guide comprising one or more guiding holes for surgical pins.
33. A kit comprising:
   a guide assembly for attaching to a patient's bone to guide bone resection procedures, the guide assembly comprises:
   an operational jig that comprises: a plurality of radio-opaque markers; one or more cutting guide slots; and one or more drill guide holes, wherein the radio-opaque markers representing the guide assembly's orientation and position with respect to the bone when viewed with an X-ray imaging tool; and
   a patient-matched guide body that comprises: a first side; a second side opposite the first side; and a guide receptacle for receiving the operational jig, wherein the second side includes at least one patient-specific surface that is configured for complementarily engaging the anatomical surface features of a target area of a patient's bone.
34. The kit of clause 33, further comprising one or more corner protectors.

## Claims

1. A surgical system comprising:
an X-ray imaging device;
a display screen; and
a computing device configured to display an X-ray image of a bone of a patient on which a patient-matched guide assembly is positioned, on the display screen;
wherein the patient-matched guide assembly includes a plurality of radio-opaque markers representing the patient-matched guide assembly's placement and alignment with respect to the bone when viewed with the X-ray imaging device;
wherein the computing device is further configured to project, on the display screen, a visual measure of quality of the patient-matched guide assembly's placement and alignment on the bone.

2. The surgical system of claim 1, wherein the patient-matched guide assembly comprises:
a patient-matched guide body with at least one patient-matched bone contacting surface;
wherein the computing device's projection of the visual measure of quality of the patient-matched guide assembly's placement and alignment on the bone comprises:
projecting an outline of the patient-matched guide body and an outline of pre-determined intended target position of the patient-matched guide body, over the X-ray image of the bone on the display screen.

3. The surgical system of any of the preceding claims, wherein the computing device's projection of the visual measure of quality of the patient-matched guide assembly's placement and alignment on the bone further comprises:
projecting a longitudinal axis of the patient-matched guide body and target longitudinal axis of the pre-determined intended target position of the patient-matched guide body, over the X-ray image of the bone on the display screen.

4. The surgical system of any of the preceding claims wherein the computing device's projection of the visual measure of quality of the patient-matched guide assembly's placement and alignment on the bone further comprises:
projecting an angular deviation between the longitudinal axis of the patient-matched guide body and the target longitudinal axis of the pre-determined intended target position of the patient-matched guide body, over the X-ray image of the bone on the display screen.

5. The surgical system of claim 2, wherein the patient-matched guide body includes a plurality of radio-opaque markers to represent the guide assembly's placement and alignment with respect to a bone when viewed with an X-ray imaging device.

6. The surgical system of claim 5, wherein the plurality of radio-opaque markers are integrally incorporated into a portion of an operational jig.

7. The surgical system of claim 6, wherein the plurality of radio-opaque markers are provided in an aligner component that is attachable to the operational jig.

8. The surgical system of claim 5, wherein the plurality of radio-opaque markers are integrally incorporated into the patient-matched guide body.

9. The surgical system of claim 2, wherein the patient-matched guide body comprises at least one patient-specific surface.

10. The surgical system of claim 6, wherein the operational jig is a resection guide comprising one or more guiding slots for bone cutting blades and the method further comprises a step of resecting the bone guided by the operational jig.

11. The surgical system of any of claims 6 and 10, wherein the operational jig is a drill guide comprising one or more guiding holes for drill bits and the method further comprises a step of drilling the bone guided by the operational jig.

12. The surgical system of any of claims 6, 10 and 11, wherein the operational jig is a pin guide comprising one or more guiding holes for surgical pins.

13. A kit comprising:
a guide assembly for attaching to a patient's bone to guide bone resection procedures, the guide assembly comprises:
an operational jig that comprises: a plurality of radio-opaque markers; one or more cutting guide slots; and one or more drill guide holes, wherein the radio-opaque markers representing the guide assembly's orientation and position with respect to the bone when viewed with an X-ray imaging tool; and
a patient-matched guide body that comprises: a first side; a second side opposite the first side; and a guide receptacle for receiving the operational jig, wherein the second side includes at least one patient-specific surface that is configured for complementarily engaging the anatomical surface features of a target area of a patient's bone.

14. The kit of claim 13, further comprising one or more corner protectors.
